# EUROPEAN PATENT SPECIFICATION

(11) **EP 3 235 386 B1**
(45) Date of publication and mention of the grant of the patent: **20.11.2024**
(21) Application number: 16166419.8
(22) Date of filing: 21.04.2016
(51) Int. Cl.: A23L 2/04, A23L 2/06, A23L 2/46, A23L 2/70, A23L 2/84, A23L 33/105, A61Q 19/00

(54) **PROCESS FOR OBTAINING JUICE FROM FRUIT PEEL**
VERFAHREN ZUR GEWINNUNG VON SAFT AUS FRUCHTSCHALEN
PROCÉDÉ D'OBTENTION DE JUS À PARTIR D'ÉPLUCHURES DE FRUITS

(43) Date of publication of application: 25.10.2017
(73) Proprietor: AMC Innova Juice & Drinks, S.L., 30100 Espinardo (ES)
(72) Inventor: GIMÉNEZ SIERRA, Teresa, 30100 ESPINARDO (ES); ARCAS MIÑARRO, Maria Cruz, 30100 ESPINARDO (ES)
(74) Representative: ZBM Patents - Zea, Barlocci & Markvardsen

(56) References cited:
- EP-A1- 2 545 898
- GB-A- 2 506 667
- US-A- 4 275 648
- US-A- 4 608 266
- US-A1- 2006 105 089
- US-A1- 2010 159 100
- US-A1- 2013 064 947
- DATABASE GNPD [online] MINTEL; July 2014 (2014-07-01), ANONYMOUS: "Yuzu & Lemon Drink", XP002762457, Database accession no. 2534863
- ANONYMOUS: "Can Citrus or OJ Scores be used to measure other juices?", 9 December 2014 (2014-12-09), pages 1, XP002762456, Retrieved from the Internet <URL:https://support.hunterlab.com/hc/en-us/articles/203566625-Can-Citrus-or-OJ-Scores-be-used-to-measure-other-juices-> [retrieved on 20160930]
- VAILLANT F ET AL: "Turbidity of pulpy fruit juice: A key factor for predicting cross-flow microfiltration performance", JOURNAL OF MEMBRANE SCIENCE, vol. 325, no. 1, 15 November 2008 (2008-11-15), ELSEVIER BV, NL, pages 404 - 412, XP025506730, ISSN: 0376-7388, [retrieved on 20080812], DOI: 10.1016/J.MEMSCI.2008.08.003

## Description

The present invention provides a method for the extraction of juice and nutritional components from fruit peel so that they can be directed to human consumption. The resulting products can be useful as a food additive in a variety of foods, juices, beverages and cosmetic products.

### BACKGROUND ART

Fruit products are now widely recognized as valuable components in a healthy diet. The most important product derived from fruits is juice. The manufacture of fruits for the production of juices generates great amounts of by-products. One of the most significant in terms of overall bulk is the peel of the fruit.

Regarding citrus fruits, the production of juice implies mainly the use of the endocarp, that is, the partitions of the fruit where most of the juice is found. The pericarp, which is composed of the albedo (mesocarp, the fruit wall) and the flavedo (exocarp, the outer peel of the fruit) are generally not used for the production of juice. Historically, the peel by-products coming from the extraction of fruit juice were neglected in terms of human consumption. At first, the by-products were simply discarded. Later, they were processed and exploited as low-value products for animal consumption such as fodder. However, the current trend is for the industry to maximize the value of the by-products generated as waste after juice extraction, i.e. to turn them as much as possible into products suitable for human consumption.

There are several interesting components present in the peel by-products of citrus fruits that, if extracted efficiently and processed properly, can become part of the human diet or can be apt for human cosmetic use. The albedo is rich in carbohydrates, pectin and dietary fiber, and the flavedo contains, apart from wax and essential oils, a series of vesicles with a high content of bitter juice. This juice, currently known in the technical field as cloudy, is of lower quality than the juice obtained from the endocarp, but can still be considered for human consumption as it contains high-value ingredients such as vitamins and natural antioxidants. Likewise, the peel of other fruits such as pomegranate, watermelon and others also contain desirable components such as vitamins, minerals and fiber.

Peel-derived juice is increasingly being used in the production of fruit juice drinks and beverages, and it is added to soft drinks and other foods to improve their nutritional content. Moreover, many of the ingredients found in the peel of a variety of fruits such as citrus fruits and pomegranate are also being directed to human use as cosmetic additives and ingredients with desirable biological properties. This demands quicker, cheaper and more efficient ways to produce peel-derived juice.

The industrial production of peel-derived juice is complicated by several factors: i) the raw juice extracted from the peel of the fruit is difficult to separate from the rest of the peel components; ii) it usually has a high content in pectins which make it very viscous, hindering its extraction and processing; iii) the stability of this juice must be ensured along its industrial processing, so that no undesirable changes in colour due to oxidation and other chemical reactions take place; iv) its bitterness must be eliminated in order to make it suitable for human consumption; v) an extra complicating issue is the fact that the viscosity, bitterness and other properties of the raw material vary not only among different types of fruits, but also among different varieties of the same fruit and even between different harvests of the same variety (ripening stage, quality, humidity),which challenge the design of a universal process for its manufacture. Thus, a single flexible process that could handle different fruits, varieties and harvests and could still deliver a final peel-derived juice within strict quality standards in terms of shelf-life, stability, viscosity and other parameters would clearly represent a step forward.

Some approaches have been disclosed in order to maximize the value of the peel components. US patent application US 2013/0064947 discloses processes for the conversion of citrus by-products for human consumption. The processes involve pressing peels to release juice from vesicles and then grinding peels in water to create a slurry. The oils are removed by a flotation technique, and the peel particles are debittered with water in steps of boiling and washing. US patent RE41537 E discloses a process for the production of refined citrus peel juice comprising a step where the raw peel juice is subjected to microfiltration to form a rentate of peel solids and a permeate of clarified peel juice, the latter being subsequently debittered.

In spite of what has been described in the art, new methods for the production of peel-derived juice that can address the mentioned issues are still being sought.

### SUMMARY OF THE INVENTION

Inventors have surprisingly found a universal procedure for the production of peel-derived juice that can be applied to a variety of fruits. Advantageously this method does not involve lengthy chemical treatments, and only entails one step of biochemical (enzymatic) treatment. The method combines a series of steps that allow it to be flexible enough to be used for a wide variety of fruits such as citric fruits, pomegranate or watermelon, but at the same time reliable enough to always give a final product meeting the highest standards.

In particular, the method is endowed with a step where a particular shredding grid is used for the initial grinding of the raw material. This shredder type has been found to give optimal results when combined with the rest of the critical steps. Further, the raw extracted juice can be initially treated with a quantity of degrading enzymes that is proportional to its initial viscosity, which results in a more controlled set of properties of the processed juice and to a more rational use of the enzymes. Moreover, the method is characterized by the addition of ascorbic and cirtic acids before the pasteurization step, which surprisingly allows having a peel derived juice that does not oxidize overly and therefore does not change in color, which is one of the problems often seen in industrial methods for obtaining juice from the fruit peel. The method can be fine-tuned so that the processed peel-derived juice can have the right values for such important properties as color (FIG.. 1), centrifugable pulp (FIG. 2), turbidity (FIG. 3) viscosity (FIG. 4) and shelf life (FIG. 5), as will be seen in the experimental data presented below.

In essence, inventors have come across a procedure made up of a series of steps that, when executed together, ensure a high quality for the final product as well as a high stability and long shelf-life.

Thus, a first aspect of the invention is a process for obtaining fruit peel juice comprising the steps of: a) Grinding of the fruit peel in an aqueous solution at 30-60 degrees Celsius with a mill comprising a shredding grid that is characterized by having an opening with circular shape from 16 to 20 mm of diameter and a surface area from 201 to 314 mm².; b) Submitting the product of step a) to an enzymatic treatment selected from the group consisting of pectinase, pectinlyase, beta-glucanase and combinations thereof; c) Separating the liquid phase from the solid phase obtained in step b); d) Sieving the liquid phase obtained in step c); and e) Adding citric acid and ascorbic acid to the liquid phase of step d); f) Pasteurizing the product obtained in step e) by thermal treatment; g) Centrifuging the product obtained in step f); provided that when the fruit is pomegranate, in step e) only citric acid is added and provided that when the fruit is lemon, in step e) no acid is added.

The juice can be successfully incorporated as an additive in a variety of final products for human consumption such as foods, mixtures with dairy products, cookies and others. It can also be incorporated as an antioxidant rich ingredient in a variety of cosmetic products.

The obtainable juice can be incorporated in a variety of drinks to increase their nutritional content.

### BRIEF DESCRIPTION OF THE DRAWINGS

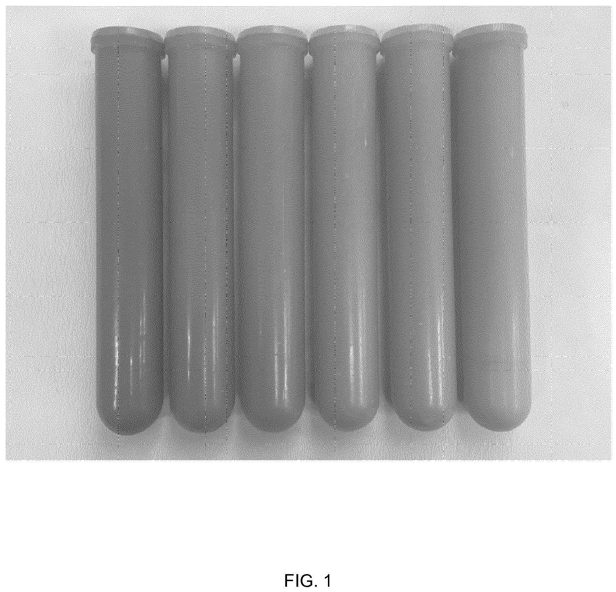

FIG.1. Tonality differences between the peel juice products obtained by method 1 (traditional process - T) and method 2 (the method of the invention - P) according to USDA color scale. The grading seen in the picture is from OJ1 (leftmost and darkest) to OJ6 (rightmost and palest). Method 1 gives tonalities of OJ1 and OJ2, whereas method 2 gives tonalities of OJ3 and OJ4.

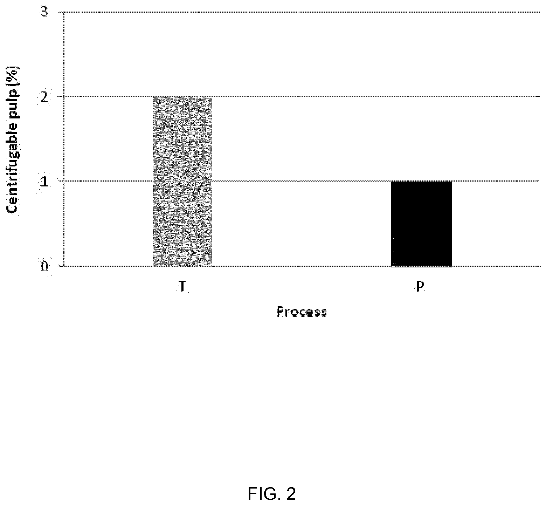

FIG.2. Centrifugable pulp differences between the fruit juice products obtained by method 1 (traditional process - T) and method 2 (the method of the invention - P) disclosed in this application.

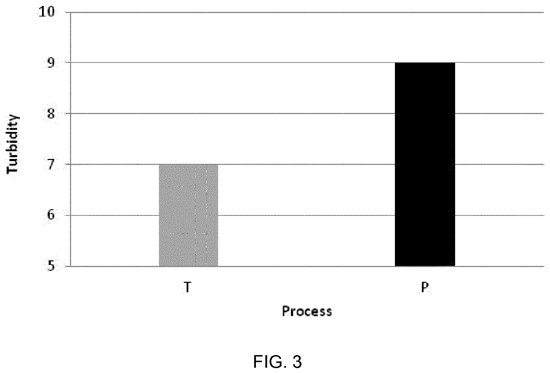

FIG.3. Turbidity differences between the fruit juice products obtained by method 1 (traditional process - T) and method 2 (the method of the invention - P) described in this application.

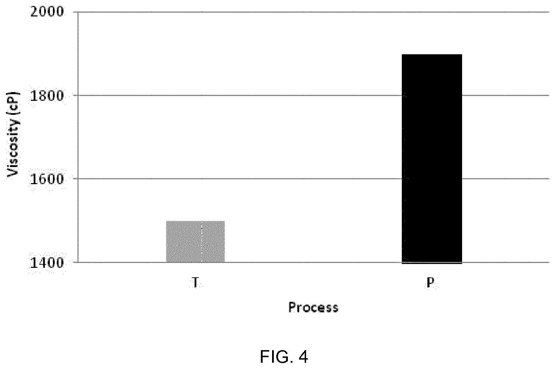

FIG.4. Viscosity differences between the fruit juice products obtained by method 1 (traditional process - T) and by method 2 (the method of the invention -P) .

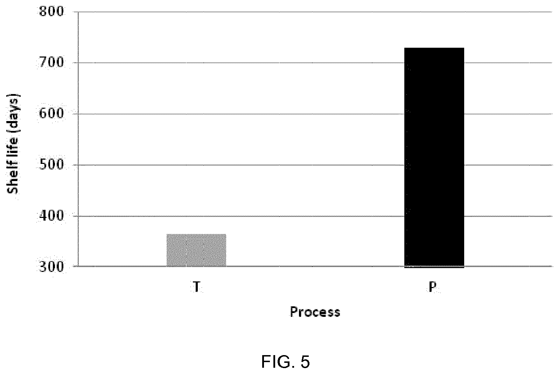

FIG.5. Shelf life differences between the fruit juice products obtained by method 1 (T) and method 2 (P).

### DETAILED DESCRIPTION OF THE INVENTION

For the sake of understanding, the following definitions are included.

The term "grinder", as used herein refers to a grinding machine for producing particle size reduction through shearing and shredding based on the use of a rotating piece armed with cutting devices that operates at very high speeds. In the case at hand, the "shredding grid" has a series of holes which effectively act as blades, cutting the fruit peel. The holes in the shredding grid can have various sizes and shapes. In the case of the present invention, the holes are circular holes of a size from 8 to 10 mm of radius (16 to 20 mm of diameter) and an area of around ("opening") 201-314mm², more preferably of 254mm².

The term "decanting" as used herein refers to a process for the separation of a mixture of two or more components by removing a layer of liquid, generally one from which a precipitate has settled. In the present application, decantation refers to separating a liquid phase containing a juice from a more solid phase containing a precipitate with solid particles.

The term "pasteurizing" as used herein refers to a process of killing bacteria in a juice or a concentrate of juice, by heating. This process is intended to kill most of the bacteria that cause spoilage, preventing the juice from losing its quality and extending its shelf-life.

The term "color" as used herein (as applied to a juice) refers to the use of the USDA scale color standard, which is applied for mandarin and orange. Currently this system is taken as a standard, and is based on 10000 different colors approximately, each one identified by a combination of a letter and a number. The result is qualitative and the observer selects the closer color of the guide which corresponds with the product [www.pantone.com].

The term "ascorbic acid" as used herein (as applied to a juice) refers to the compound whose IUPAC name is (5*R*)-[(1*S*)-1,2-Dihydroxyethyl]-3,4-dihydroxyfuran-2(5*H*)-one. The method used for the determination of total ascorbic acid in the fruit juice was as specified in the standard AOAC methods, 939.13 and 966.18. This method was chosen due to its advantages compared to other methods. It is sensitive, economic, practical and less time-consuming [JBT FoodTech Citrus Systems, 2011a; AOAC, 1999; Food Chemicals Codex. 1996].

The term "centrifugable pulp" as used herein (as applied to a juice) refers to the percentage of pulp sedimented after centrifugation at 370g during 10 minutes. The method used for the determination centrifugable pulp is described in UNE-EN 12134:1998. (AENOR, 1998, IFUMA60, 1998). The results were determined at the end of the production process and during the time of cloudy storage.

The term "viscosity" as used herein (as applied to a juice) refers to the measure of the resistance the juice opposes to deformation. It corresponds to the informal concept of "thickness". Of note, the methodology used in the present application to determine the viscosity is performed using a Brookfield^{®} viscometer and following the method described in the Laboratory manual of JBT FoodTech Citrus Systems (JBT FoodTech Citrus Systems, 2011b)
The term "turbidity" as used herein refers to the cloudiness or haziness of the juice caused by large numbers of individual particles that are typically invisible to the naked eye. Of note, the methodology used in the present application to determine the turbidity is performed by the previous dilution of samples of cloudy (at 5%) and a second step of centrifugation (1500 rpm, 20 min). After that, determination of solution turbidity is performed following the spectrophotometrical method of attenuation based on Beer's law, which explains the relationship between transmitted and Incident lights. For the analysis distilled water as blank, optical glass cuvettes of 1 cm thickness and a wavelength of 280 nm were used. (IFUREC07, 2003; EPA, 1999, Anderson, 1995).

The term "stability" as used herein refers to the time (days) which the cloudy remains without phase separation or without clarification and is determined by visual observations along the storage time.

The term "Brix index" as used herein refers to the sugar content in an aqueous solution. One degree Brix is 1 gram of sucrose in 100 grams of solution.

The term "debittering step" as used herein refers to a step where the product is treated so that the compounds that give it a bitter taste are eliminated. For instance, it is common practice to use the enzyme naringinase. It is a debittering enzyme that is used in the commercial production of citrus beverages and juices. It breaks down the compound naringin that gives citrus juices its bitter taste.

The term "stone fruit" as used herein refers to a fruit in which an outer fleshy part (exocarp, or skin; and mesocarp, or flesh) surrounds a shell (the pit, stone, or pyrene) of hardened endocarp with a seed (kernel) inside. The crucial characteristic of a drupe is that the hard, "lignified" stone (or pit) is derived from the ovary wall of the flower. Typical stone fruits include apricot, cherry, nectarine, peach or plum.

As mentioned above, a first aspect of the invention is a process for obtaining fruit peel juice comprising the steps of: a) Grinding of the fruit peel in an aqueous solution at 30-60 degrees Celsius with a mill comprising a shredding grid that is characterized by having an opening with circular shape from 16 to 20 mm of diameter and a surface area from 201 to 314 mm²; b) Submitting the product of step a) to an enzymatic treatment selected from the group consisting of pectinase, pectinlyase, beta-glucanase, cellulase and combinations thereof; c) Separating the liquid phase from the solid phase obtained in step b); d) Sieving the liquid phase obtained in step c); and e) Adding citric acid and ascorbic acid to the liquid phase of step d); f) Pasteurizing the product obtained in step e) by thermal treatment; g) Centrifuging the product obtained in step f); provided that when the fruit is pomegranate, in step e) only citric acid is added and provided that when the fruit is lemon, in step e) no acid is added.

In an embodiment of the first aspect of the invention, in step c) the separation of the liquid phase from the solid phase is carried out by decanting.

In an embodiment of the first aspect of the invention, the grinding of the fruit peel in an aqueous solution is carried out at 20 to 55 degrees Celsius.

In another embodiment of the first aspect of the invention, in step a) the mill comprises a shredding grid that is characterized by having an opening with circular shape.

In another embodiment of the first aspect of the invention, in step a) the mill comprises a shredding grid that is characterized by having an opening with circular shape and a surface area (opening) of 254mm².

In another embodiment of the first aspect of the invention, the process further comprises a step h) characterized by concentrating the liquid phase obtained in step g) and wherein the Brix index of the processed peel juice is from 40 to 65.

In another embodiment of the first aspect of the invention, the process further comprises a step h) characterized by concentrating the liquid phase obtained in step g) and wherein the Brix index of the processed peel juice is from 50 to 60.

In another embodiment of the first aspect of the invention, the process has an efficiency such that from 10 to 30 metric tons of preconcentrated cloudy, 1 metric ton of final cloudy is obtained.

In another embodiment of the first aspect of the invention, in step b) the amount of pectinase added is from 1 to 2800 ppm, the amount of pectinlyase added is from 1 to 2800 ppm, the amount of beta-glucanase added is from 1 to 1800 ppm, and the amount of cellulase added is from 10 to 600 ppm.

In another embodiment of the first aspect of the invention, in step b) the amount of enzyme added is directly proportional to the viscosity of the product obtained in step a).

In another embodiment of the first aspect of the invention, in step b) the amount of enzyme added is directly proportional to the viscosity of the product obtained in step a), and said viscosity is controlled by a viscosimeter deployed in-line which allows monitoring the viscosity continuously. In other words, the viscosity of the product resulting from step a) dictates the amount of enzyme added in step b), such that when the viscosity is higher, a higher amount of enzyme is added.

In another embodiment of the first aspect of the invention, in step b) the treatment is administered from 15 to 90 minutes and the temperature at which it is carried out is from 20 to 55 degrees Celsius

In another embodiment of the first aspect of the invention, in step b) the amount of pectinase added is from 1 to 2800 ppm, the amount of pectinlyase added is from 1 to 2800 ppm, the amount of beta-glucanase added is from 1 to 1800 ppm, and the amount of cellulase added is from 10 to 600 ppm, and wherein the treatment is administred from 15 to 90 minutes and the temperature at which it is carried out is from 20 to 55 degrees Celsius

In another embodiment of the first aspect of the invention, in step b) the amount of pectinase added is from 1 to 2800 ppm, the amount of pectinlyase added is from 1 to 2800 ppm, the amount of beta-glucanase added is from 1 to 1800 ppm, and the amount of cellulase added is from 10 to 600 ppm, and wherein the treatment is administred from 25 to 80 minutes and the temperature at which it is carried out is from 20 to 55 degrees Celsius

In another embodiment of the first aspect of the invention, in step b) the amount of pectinase added is from 1 to 2800 ppm, the amount of pectinlyase added is from 1 to 2800 ppm, the amount of beta-glucanase added is from 1 to 1800 ppm, and the amount of cellulase added is from 10 to 600 ppm, and wherein the treatment is administred from 35 to 70 minutes and the temperature at which it is carried out is from 20 to 55 degrees Celsius

In another embodiment of the first aspect of the invention, in step b) the amount of pectinase added is from 1 to 2800 ppm, the amount of pectinlyase added is from 1 to 2800 ppm, the amount of beta-glucanase added is from 1 to 1800 ppm, and the amount of cellulase added is from 10 to 600 ppm, and wherein the treatment is administred from 45 to 60 minutes and the temperature at which it is carried out is from 20 to 55 degrees Celsius

In another embodiment of the first aspect of the invention, the process further comprises a step i) wherein the product obtained in steps g) or h) is pasteurized by thermal treatment.

In another embodiment of the first aspect of the invention, the process for obtaining fruit peel juice further comprises a step i) comprising pasteurizing the product obtained in steps g) or h) by thermal treatment.

In another embodiment of the first aspect of the invention, step e) comprises adding citric acid together with ascorbic acid to adjust the pH to a value from 3.2 to 4.2 and a final concentration from 250 to 450 mg/kg.

In another embodiment of the first aspect of the invention, the process further comprises an additional debittering step between steps g) and h). In another embodiment of the first aspect of the invention, the debittering comprises a treatment characterized by the absorption of the compounds that confer bitterness in a column.

The product "obtainable by" the process of the invention is used here to define the product by the process for obtaining it. For the purposes of the invention the expressions "obtainable", "obtained" and equivalent expressions are used interchangeably, and in any case, the expression "obtainable" encompasses the expression "obtained".

Throughout the description and claims the word "comprise" and variations of the word, are not intended to exclude other technical features, additives, components, or steps. Furthermore, the word "comprise" and its variations encompasses the term "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### EXAMPLES

In order to prove that the process for obtaining fruit peel juice of the present invention is superior to very closely related methods, it was decided to run a series of comparative experiments. The fruit peel juice obtained by the invention was compared to the fruit peel juice of a very closely related method. As it can be seen from the data presented below, the method of the invention (method 2) unexpectedly outperforms the closely related method (method 1) in terms of turbidity, viscosity, color and stability of the final product.

### A) Methods

### Method 1.

1. The process to obtain a fruit peel juice began with the grinding step of the fruit peel in an aqueous solution at 50 degrees Celsius by using a grid with a rectangular shape with a surface of 100 mm2. The crushed peels were mixed with water in a blending tank.
2. The result of step 1 was sent to a reactor tank where the product was submitted to an enzymatic treatment with pectinlyase in a concentration of 2 mg/L, for a period of time of 30 minutes. The grinded peel was kept in the reaction tanks during certain time depending of the fruit type, the maturity index and the moment of the season, in order to carry out the optimal reaction to continue with the process.
3. The next step was a decantation to separate the liquid phase from the solid one of the mix of peel juice and peels. This step was carried out with the help of a high speed decanter (Gea Wesfalia).
4. The obtained product was sent to a Vibro-Energy Round Separator Swueco. This was an additional separation step but more restrictive where coarser particles were removed by screen vibration.
5. The liquid obtained after these two separation steps was kept in storage tanks, in order to collect enough volume to proceed with the next step.
6. The product was treated by heat in a plates pasteurizer in order to inactivate the enzymes added in the second step. This heat treatment was at 50 ° C during 60 seconds.
7. After that, the pasteurized product was passed through a centrifugal separator to reduce centrifugable pulp below 1 %.
8. After that as optional step, depending on the final requirement for the products obtained, the low pulp liquid was de-bittered by absortion column to retain polyphenols and bitter molecules (e.g. limonin in citrus).
9. The next step was an evaporation process for concentrating the fruit peel juice. The evaporation was carried out by a 'Wiegand Technology with 5 effects'. The product was evaporated until 50 ° Brix was attained.
10. The product was cooled in a cooling tower and after that stored in a tank before final storage.
11.The frozen product was filled into drums and sent to the camera to reach - 20 ° C.
12. The aseptic product was sent to a tubular pasteurizer and treated at 95 ° C during 60 seconds depending of the product. After that the product was preserved at cold temperatures (lower than 4° C).

### Method 2.

This method falls within the definition of claim 1 as it is set out in the present application, and is characterized by:
1. The process to obtain a fruit peel juice started with the grinding step of the fruit peel in an aqueous solution at 50 degrees Celsius by using a grid with a circular shape of 18 mm of diameter and a surface area of 254 mm2. The crushed peels were mixed with water in a blending tank.
2. The result of step 1 was sent to a reactor tank where the product was submitted to an enzymatic treatment with pectinlyase. in a concentration of 2 mg/L, for a period of time of 30 minutes in order to carry out the optimal reaction to continue with the process.
3. The next step was a decantation to separate the liquid phase from the solid one of the mix of peel juice and peels. This step was carried out by a high speed decanter (Gea Wesfalia).
4. The obtained product was sent to a Vibro-Energy Round Separator Swueco. This was an additional separation step but more restrictive where coarser particles are removed by screen vibration.
5. The liquid obtained after these two separation steps was kept in storage tanks, in order to collect enough volume to go to the next step.
6. Ascorbic (0.0012 Kg of ascorbic acid per Kg of cloudy) and citric acid (0.0248 Kg of citric acid per Kg of cloudy) were added to the liquid obtained until adjustement of the pH to 3.5 ±0.5
7. The product was treated by heat in order to inactivate the enzymes added in the second step. This heat treatment was at 50 ° C during 60 seconds.
8. After that, the pasteurized product was passed through a centrifugal separator to reduce centrifugable pulp below 1 %.
9. After that as optional step, depending of the final requirement of the product, the low pulp liquid was de-bittered by absortion column to retain polyphenols and bitter molecules (e.g. limonin in Citrus).
10. The next step was an evaporation process for concentrating the fruit peel juice, the evaporation was done by a 'Wiegand Technology with 5 effects'. The product was evaporated until a 50 ° Brix was attained.
11. The product was cooled in a cooling tower and after that, it was stored in a tank before final storage, which could be frozen or aseptic.
12. The frozen product was filled in drums and sent to the camera to reach -20 ° C.
13. The aseptic product was sent to a tubular pasteurizer and treated at 95 ° C during 60 seconds depending of the product. After that the product is preserved at cold temperatures (lower than 4° C).

### B) Results

Method 1, that is, a process for producing fruit peel juice that is very closely related, but different, to the method of the invention, gave a fruit peel juice obtained showing a dark yellow color classified in the USDA color scale as OJ1 and OJ2 (FIG.1), centrifugable pulp of 2 % (FIG.2), turbidity value below 7.0 (FIG.3), viscosity of 1500 centiPoisses (cPs) (FIG.4) and a maximum shelf life of 365 days (FIG.5). In addition the obtained fruit peel juice according to this procedure gave a phase separation due to stability loss, with a little dark yellow tonality and the preservation of sensory properties was kept for a maximum of 365 days.

In contrast, method 2, that is, the method of the invention, gave a fruit peel juice showing a better color, classified as OJ3 and OJ4 in the USDA colour scale (FIG.1), centrifugable pulp of 1.0 % (FIG.2), turbidity value of 9.0 (FIG.3), viscosity of 1900 cP (FIG.4 ) and a shelf life of 730 days (FIG.5). These properties improved the quality of the fruit peel juice as a semi-final product for a variety of applications. The juice obtained was less prone to phase separation, had a better yellow tonality and the preservation of sensory properties was up to 730 days.

### REFERENCES CITED IN THE APPLICATION

Cioffi, M., et. al. "Serum-soluble E-cadherin fragments in lung cancer", Tumori 1999, vol. 85, pp. 32-34
US 2013/0064947
US patent RE41537 E
www.pantone.com
JBT FoodTech Citrus Systems, 2011a. 27. Ascorbic Acid by Iodine Titration.
Laboratory Manual. PROCEDURES FOR ANALYSIS OF CITRUS PRODUCTS, 6th Ed., pp 88-90. Manual No. 054R10020.000-6 Copyright 2011 by John Bean Technologies Corporation, Inc., Florida, USA.
AOAC, 1999. Official methods of analysis of the Association of official Analytical chemist. 16th edition, 5th revision. AOAC international, Gaithersburg, M.D. method 939.13 & 966.18
Food Chemicals Codex. 1996. 14th ED., Food and Nutrition Board Institute of Medicine National Academy of Sciences, National Academy Press, Washington, D.C., p. 33.
AENOR, 1998. UNE-EN 12134:1998 Fruit and vegetable juices. Determination of centrifugable pulp content.
IFUMA 60. (1998). International Federation Fruit Juice Producers methods. revised 2005.
JBT FoodTech Citrus Systems, 2011b. Chapter 9. Viscosity (Using Standard Spindle). Laboratory Manual. PROCEDURES FOR ANALYSIS OF CITRUS PRODUCTS, 6th Ed., pp 38-40. Manual No. 054R10020.000-6 Copyright 2011 by John Bean Technologies Corporation, Inc., Florida, USA. IFUREC07. 2003. Recommendations for Turbidity Measurements. International Federation Fruit Juice Producers methods.
EPA. (1999). Chapter 11. Basic Turbidimeter Design and Concepts. In Guidance Manual for Compliance with the Interim Enhanced Surface Water Treatment Rule: Turbidity Provisions. N.p.: US Government Printing Office. http://www.epa.gov/ogwdw/mdbp/pdf /turbidity/chap_11.pdf
Anderson, C. W. (2005). Turbidity 6.7. In USGS National Field Manual for the Collection of Water-Quality Data. U S Geological Survey.

## Claims

1. A process for obtaining fruit peel juice comprising the steps of:
a) Grinding of the fruit peel in an aqueous solution at 30-60 degrees Celsius with a mill comprising a shredding grid that is **characterized by** having an opening with circular shape from 16 to 20 mm of diameter and a surface area from 201 to 314 mm²;
b) Submitting the product of step a) to an enzymatic treatment selected from the group consisting of pectinase, pectinlyase, beta-glucanase, cellulase and combinations thereof;
c) Separating the liquid phase from the solid phase obtained in step b);
d) Sieving the liquid phase obtained in step c); and
e) Adding citric acid and ascorbic acid to the liquid phase of step d);
f) Pasteurizing the product obtained in step e) by thermal treatment;
g) Centrifuging the product obtained in step f);
provided that when the fruit is pomegranate, in step e) only citric acid is added and provided that when the fruit is lemon, in step e) no acid is added.

2. The process for obtaining fruit peel juice of claim 1 further comprising the step:
h) Concentrating the liquid phase obtained in step g) and wherein the Brix index is from 40 to 65.

3. The process for obtaining fruit peel juice of any one of claims 1-2 wherein in step b), the amount of pectinase added is from 1 to 2800 ppm, the amount of pectinlyase added is from 1 to 2800 ppm, the amount of beta-glucanase added is from 1 to 1800 ppm, and the amount of cellulase added is from 10 to 600 ppm, and wherein the treatment is administred from 15 to 90 minutes and the temperature at which it is carried out is from 20 to 55 degrees Celsius..

4. The process for obtaining fruit peel juice of any one of claims 1-3 further comprising a step i) comprising pasteurizing the product obtained in steps g) or h) by thermal treatment.

5. The process for obtaining fruit peel juice of any one of claims 1-4, wherein step e) comprises adding citric acid together with ascorbic acid to adjust the pH to a value from 3.2 to 4.2 and a final concentration from 250 to 450 mg/kg.

6. The process for obtaining fruit peel juice of any one of claims 1-5, further comprising an additional debittering step between steps g) and h).

## Patentansprüche

1. Ein Verfahren zum Erhalten von Fruchtschalensaft, umfassend die folgenden Schritte:
a) Mahlen der Fruchtschale in einer wässrigen Lösung bei 30-60 Grad Celsius mit einer Mühle, die ein Zerkleinerungsgitter umfasst, das **dadurch gekennzeichnet ist, dass** es eine kreisförmige Öffnung von 16 bis 20 mm Durchmesser und einen Oberflächenbereich von 201 bis 314 mm² hat;
b) Unterziehen des Produkts von Schritt a) einer enzymatischen Behandlung, die ausgewählt ist aus der Gruppe bestehend aus Pektinase, Pektinlyase, Beta-Glucanase, Cellulase und Kombinationen davon;
c) Abtrennen der flüssigen Phase von der in Schritt b) erhaltenen festen Phase;
d) Sieben der in Schritt c) erhaltenen flüssigen Phase; und
e) Zugabe von Zitronensäure und Ascorbinsäure zur flüssigen Phase von Schritt d);
f) Pasteurisieren des in Schritt e) erhaltenen Produkts durch thermische Behandlung;
g) Zentrifugieren des in Schritt f) erhaltenen Produkts;
unter der Voraussetzung, dass, wenn es sich bei der Frucht um Granatapfel handelt, in Schritt e) nur Zitronensäure zugegeben wird und unter der Voraussetzung, dass, wenn es sich bei der Frucht um Zitrone handelt, in Schritt e) keine Säure zugesetzt wird.

2. Das Verfahren zur Gewinnung von Fruchtschalensaft von Anspruch 1, weiterhin umfassend den Schritt:
h) Konzentrieren der in Schritt g) erhaltenen flüssigen Phase, wobei der Brix-Index 40 bis 65 beträgt.

3. Das Verfahren zum Erhalten von Fruchtschalensaft von einem der Ansprüche 1 bis 2, wobei in Schritt b) die Menge der zugegebenen Pektinase von 1 bis 2800 ppm ist, die Menge der zugegebenen Pektinlyase von 1 bis 2800 ppm ist, die Menge der zugegebenen Beta-Glucanase von 1 bis 1800 ppm ist und die Menge der zugegebenen Cellulase von 10 bis 600 ppm ist, und wobei die Behandlung von 15 bis 90 Minuten angewendet wird und die Temperatur, bei der sie durchgeführt wird, von 20 bis 55 Grad Celsius ist.

4. Das Verfahren zur Gewinnung von Fruchtschalensaft von einem der Ansprüche 1 bis 3, das ferner einen Schritt i) umfasst, der das Pasteurisieren des in den Schritten g) oder h) erhaltenen Produkts durch thermische Behandlung umfasst.

5. Das Verfahren zur Herstellung von Fruchtschalensaft nach einem der Ansprüche 1 bis 4, wobei der Schritt e) die Zugabe von Citronensäure zusammen mit Ascorbinsäure umfasst, um den pH-Wert auf einen Wert von 3,2 bis 4,2 und eine Endkonzentration von 250 bis 450 mg/kg einzustellen.

6. Das Verfahren zum Erhalten von Fruchtschalensaft von einem der Ansprüche 1 bis 5, ferner umfassend einen zusätzlichen Entbitterungsschritt zwischen den Schritten g) und h).

## Revendications

1. Un procédé pour obtenir du jus d'écorce de fruit comprenant les étapes suivantes :
a) Broyage de l'écorce de fruit dans une solution aqueuse à 30-60 degrés Celsius avec un broyeur comprenant une grille de déchiquetage qui est **caractérisée par** une ouverture de forme circulaire de 16 à 20 mm de diamètre et une aire de surface de 201 à 314 mm² ;
b) Soumettre le produit de l'étape a) à un traitement enzymatique choisi dans le groupe constitué par la pectinase, la pectinlyase, la bêta-glucanase, la cellulase et des combinaisons de celles-ci ;
c) Séparer la phase liquide de la phase solide obtenue dans l'étape b) ;
d) Tamiser la phase liquide obtenue dans l'étape c) ; et
e) Ajouter de l'acide citrique et de l'acide ascorbique à la phase liquide de l'étape d) ;
f) Pasteuriser le produit obtenu dans l'étape e) par traitement thermique ;
g) Centrifuger le produit obtenu dans l'étape f) ;
à condition que lorsque le fruit est la grenade, dans l'étape e) seul de l'acide citrique soit ajouté et à condition que lorsque le fruit est le citron, dans l'étape e) aucun acide ne soit ajouté.

2. Le procédé pour obtenir du jus d'écorce de fruit de la revendication 1, comprenant en outre l'étape :
h) Concentrer la phase liquide obtenue dans l'étape g) et dans laquelle l'indice Brix est de 40 à 65.

3. Le procédé pour obtenir du jus d'écorce de fruit de l'une quelconque des revendications 1 à 2, dans lequel dans l'étape b), la quantité de pectinase ajoutée est de 1 à 2800 ppm, la quantité de pectinlyase ajoutée est de 1 à 2800 ppm, la quantité de bêta-glucanase ajoutée est de 1 à 1800 ppm, et la quantité de cellulase ajoutée est de 10 à 600 ppm, et dans lequel le traitement est administré de 15 à 90 minutes et la température à laquelle il est effectué est de 20 à 55 degrés Celsius.

4. Le procédé pour obtenir du jus d'écorce de fruit de l'une quelconque des revendications 1 à 3, comprenant en outre une étape i) comprenant la pasteurisation du produit obtenu dans les étapes g) ou h) par traitement thermique.

5. Le procédé pour obtenir du jus d'écorce de fruit de l'une quelconque des revendications 1 à 4, dans lequel l'étape e) comprend ajouter de l'acide citrique avec de l'acide ascorbique pour ajuster le pH à une valeur de 3,2 à 4,2 et une concentration finale de 250 à 450 mg/kg.

6. Le procédé d'obtention de jus d'écorce de fruit de l'une quelconque des revendications 1 à 5, comprenant en outre une étape supplémentaire de désamérisation entre les étapes g) et h).
